# EUROPEAN PATENT APPLICATION

(11) **EP 3 456 701 A1**
(43) Date of publication of application: **20.03.2019**
(21) Application number: 17191887.3
(22) Date of filing: 19.09.2017
(51) Int. Cl.: C07B 61/00, C07D 233/84, C07D 235/28, C07D 473/12

(54) **METHOD FOR ALKYLATING A MOLECULE OR AN ION**

(71) Applicant: Max-Planck-Gesellschaft zur Förderung der Wissenschaften e.V., 80539 München (DE)
(72) Inventor: ANTONIETTI, Markus, 14558 Nuthetal (DE); GUTERMAN, Ryan, Thornhill L3T7H9 Ontario (CA); MIAO, Han, 355200 Fuding, Fujian (CN)
(74) Representative: Henkel, Breuer & Partner

(57) **Abstract**

The present invention relates to a method for alkylating a molecule or an ion, wherein the molecule or the ion is alkylated by reacting it with an alkylating agent, wherein the alkylating agent is a compound according to formula (1). In the formula (1), R¹ is a substituted or unsubstituted C₁₋₂₀ hydrocarbon residue or a polymer, R² is a substituted or unsubstituted C₁₋₂₀ hydrocarbon residue or a hydrogen atom, R³ is absent, a substituted or unsubstituted C₁₋₂₀ hydrocarbon residue or a hydrogen atom, R⁴ and R⁵ are linked with each other to form an aromatic group or are independently from each other selected from the group consisting of a hydrogen atom, substituted and unsubstituted C₁₋₂₀ hydrocarbon residues and electron withdrawing groups, X¹ is an oxygen atom, a nitrogen atom or a sulfur atom, wherin R³ is not present in formula (1) if X¹ is an oxygen atom or a sulfur atom, Z is a covalent bond or an aromatic group and A⁻ is an anion.

## Description

The present invention relates to a method for alkylating a molecule or an ion as well as to a compound suitable as alkylating agent.

Alkylation is a reaction, in which an alkyl group is transferred from a first molecule or ion to a second molecule or ion. The first molecule or ion, from which the alkyl group is transferred to the second molecule or ion, is called alkylating agent. Alkylation is a very important reaction in chemistry and biology, why alkylation agents are not only important commercial products, but are also widely present in nature. In nature, for instance alkylation agents are important for the synthesis and manipulation of a variety of biomolecules, including proteins, DNA, RNA, peptides, and carbohydrates. A prominent example for alkylation in nature is the methylation of DNA, during which methyl groups are transferred by enzymes to the bases of the DNA. DNA methylation is supposed to occur in any animate being.

Moreover, alkylating agents are used in the chemical industry, pharmaceutic industry and medicine. For instance, some chemotherapeutic agents are alkylation agents, namely the so called alkylating antineoplastic agents, which attaches alkyl groups to the number seven nitrogen atom of the purine ring of the guanine base of the DNA. The positive function of the alkylating antineoplastic agents as chemotherapeutic agents is due to the fact that cancer cells, in general, proliferate faster and with less error-correcting than healthy cells. On account therefore, cancer cells are more sensitive to the DNA damage resulting from the alkylation initiated by the alkylating antineoplastic agents.

In addition, alkylation of peptides is used in the pharmaceutic industry for example for peptide mapping or for inhibition of peptides. Alkylation reactions are also important in organic synthesis, for example for alkylating amines or as reagents in the Williamson ether synthesis and in the Stork enamine alkylation.

The known alkylating agents used to date, are not completely satisfactory, but are connected with disadvantages. The vast majority of the alkylating agents are known carcinogens and often have a high vapor pressure, i.e. these alkylating agents often are highly volatile at the alkylation temperature, i.e. the temperature at which they are used during the alkylation reaction. For example, dimethyl-sulphate is a cheap and easily produced methylating agent, however it is extremely toxic, corrosive and carcinogenic, and even considered to be a chemical weapon. Other reagents such as iodomethane, triethyloxonium salts and methyltriflate are comparably less toxic, but are still powerful carcinogens having a high vapor pressure at the alkylation temperature and often are not stable in air and water. As a result, they can easily be inhaled during the alkylation by the operators. In addition, for safe storage without risk of explosion security measures are required.

Chiappe et al. describe in Green Chemistry, 2006, pages 277 to 281 a method for the selective preparation of N-monoalkyl-substituted anilines employing ionic liquids as a solvent. Alkyl, allyl and benzyl halides serve as alkylating agents to alkylate differently substituted anilines as substrates. It was observed that the ionic liquid may promote the alkylation of aromatic amines, but also reduces or eliminates the formation of overalkylation products. Nevertheless, although this method may also be applied for synthesizing more complex structures, as described by Monopoli et al. in European Journal of Organic Chemistry 2012, pages 3105 to 3111, the method is based on alkyl halides, i.e. on conventional alkylating agents having the above described drawbacks.

In US 8,722,943 B1 a method is disclosed, in which hydroxyl moieties of a polyol are alkylated using alkylating agents for producing alkoxy polyol ethers. Alkyl tosylates are mentioned as suitable alkylating agents and tosylate moieties may be linked to a resin, such as Dowex 50W. However, the alkyl tosylates do not provide many possibilities for controlling their reactivity and therefore have a limited versatility.

In view of the above, the object underlying the present invention is to provide a method for alkylating a molecule, which is safe, which is without health concern for the operators and which is effective.

In accordance with the present invention, this object is satisfied by a method for alkylating a molecule or an ion, wherein the molecule or the ion is alkylated by reacting it with an alkylating agent, wherein the alkylating agent is a compound according to formula (1): 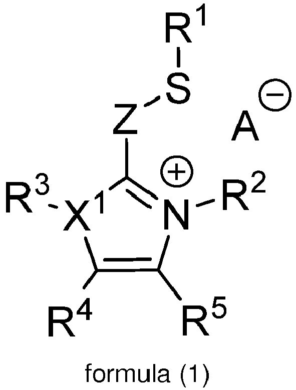 wherein in formula (1) R¹ is a substituted or unsubstituted C₁₋₂₀ hydrocarbon residue or a polymer, R² is a substituted or unsubstituted C₁₋₂₀ hydrocarbon residue or a hydrogen atom, R³ is absent, a substituted or unsubstituted C₁₋₂₀ hydrocarbon residue or a hydrogen atom, R⁴ and R⁵ are linked with each other to form an aromatic group or are independently from each other selected from the group consisting of a hydrogen atom, substituted and unsubstituted C₁₋₂₀ hydrocarbon residues and electron withdrawing groups, X¹ is an oxygen atom, a nitrogen atom or a sulfur atom, wherein R³ is not present in formula (1) if X¹ is an oxygen atom or a sulfur atom, Z is a covalent bond or an aromatic group and A⁻ is an anion.

This solution bases on the surprising finding that compounds according to the formula (1) are not only very effective alkylating agents, since the alkylation residue R¹ is effectively transferred during the alkylation reaction to the molecule or ion to be alkylated due to the comparable weak bond between the sulfur atom and the residue R¹, but that these compounds also have a comparable low vapor pressure at the alkylation temperature. Therefore, these compounds do not or at least not significantly produce vapor during the alkylation. Therefore, they are without or at least with at most low health concerns for the operators due to the low risk of being inhaled, but nevertheless very effective. A further advantage of the aforementioned compounds is that they can be easily derivatized at the residues R² to R⁵, Z and X¹ and varied by selecting an appropriate anion A⁻, so that the reactivity and solubility of the compounds can be tailored to the needs of the specific alkylation reaction. Therefore, the method in accordance with the present invention is versatile. Moreover, these compounds are stable in air and water so as to allow to be safely stored without risk of explosion. All in all, the method in accordance with the present invention is safe, has no or at least no significant health concerns and is effective.

Electron withdrawing groups are according to the present invention groups that draw electron density from neighboring groups. This effect can be calculated via quantum chemical methods or observed, for example by nuclear magnetic resonance spectroscopy.

Preferably, in formula (1) at least one of R⁴ and R⁵ is an electron withdrawing group which is selected from the group consisting of carbonyl groups, hydroxyl groups, halogen atoms, carboxy groups, cyano groups fluorinated and perfluorinated alkyl groups. The provision of such electron withdrawing groups draws electrons from the five-membered aromatic ring system and thus weakens the strength of the bond between the sulfur atom and residue R¹, why the residue R¹ may be more easily transferred during the alkylation reaction.

Good results are in particular obtained, when at least one of R⁴ and R⁵ is a substituted and unsubstituted C₁₋₂₀ hydrocarbon residue, preferably a substituted and unsubstituted C₁₋₁₀ hydrocarbon residue and more a C₁₋₆ hydrocarbon residue.

It is further preferred that Z is a covalent bond, i.e. that the five-membered aromatic ring is directly bound to the sulfur atom.

In a further development of the idea of the present invention, it is proposed that X¹ is a nitrogen atom. This forms a particular stable five-membered aromatic ring.

In particular preferred is that at least one of R⁴ and R⁵ is an electron withdrawing group which is selected from the group consisting of carbonyl groups, hydroxyl groups, halogen atoms, carboxy groups, cyano groups fluorinated and perfluorinated alkyl groups, that Z is a covalent bond and that X¹ is a nitrogen atom.

Subsequently, three particular preferred embodiments of the method in accordance of the present invention are described, wherein also preferred embodiments for the aforementioned formula (1) are described in this description.

First particular preferred embodiment of the method of the present invention

According to a first particular preferred embodiement of the present invention, the residues R⁴ and R⁵ in formula (1) are hydrogen atoms.

Thus, in accordance with the first particular preferred embodiement of the present invention the method for alkylating a molecule or an ion comprises the step of reacting the molecule or the ion to be alkylated with an alkylating agent, wherein the alkylating agent is preferably a compound according to formula (2): 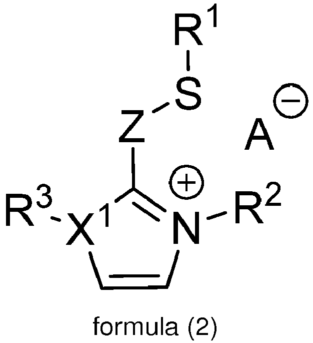 wherein residues R¹, R², R³, X¹, Z and A⁻ are defined as described above for the general formula (1). The use of a compound according to the formula (2) as alkylating agent allows - in addition to the aforementioned advantages - to very easily remove byproduct. Furthermore, the compound may be easily synthesized, is highly stability and has a comparable low reactivity for higher-temperature applications.

It is preferred that Z in formula (2) is a covalent bond.

It is also preferred that X¹ in formula (2) is a nitrogen atom.

In a further preferred embodiment, Z is a covalent bond and X¹ is a nitrogen atom.

Preferably, in formulae (1) and (2) R² and R³ are independently from each other a substituted or unsubstituted C₁₋₂₀ hydrocarbon residue. It is further preferred that R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. Even more preferably, R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group. Most preferably, R² and R³ are independently from each other a substituted or unsubstituted methyl group, ethyl group, phenyl group, or benzyl group. The reactivity of the compound increases, from least reactive to most reactive, from alkyl to benzyl to alkenyl to phenyl.

In accordance with still a further preferred embodiment, R² and R³ in formula (1) and/or in formula (2) are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, Z is a covalent bond and X¹ is a nitrogen atom.

It is even more preferred that R² and R³ in formula (1) and/or in formula (2) are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group, Z is a covalent bond and X¹ is a nitrogen atom. Most preferably, R² and R³ are independently from each other a substituted or unsubstituted methyl group, ethyl group or benzyl group, Z is a covalent bond and X¹ is a nitrogen atom.

In yet a further preferred embodiment, in formulae (1) and (2) R² is equal to R³. If R² is equal to R³ synthesis of the compounds according to formulae (1) and (2) is even easier than in the case that both residues are different to each other. With this respect, it is particularly preferred in formula (1) and particularly in formula (2) when R² is equal to R³, Z is a covalent bond and X¹ is a nitrogen atom.

In formulae (1) and (2) R¹ is preferably a substituted or unsubstituted C₁₋₂₀ hydrocarbon residue. It is even more preferred that R¹ contains at least one of a hydroxy group and/or an amine group. Some examples for R¹ are a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. It is particularly preferred that R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group. Even more preferably, R1 is a substituted or unsubstituted C₁₋₆ alkyl group, a substituted or unsubstituted C₄₋₈ cycloalkyl group, a substituted or unsubstituted C₂₋₆ alkenyl group or a substituted or unsubstituted C₂₋₆ alkinyl group. Most preferably, R¹ is a methyl group or an ethly group.

Alternatively, R¹ in formulae (1) and (2) is a polymer having a molecular weight of 150 to 500000 g/mol, preferably from 500 to 5000 g/mol. It is particularly preferred that R¹ is a synthetic polymer and even more preferred that R¹ is a synthetic polymer having a molecular weight of 150 to 500000 g/mol, preferably from 500 to 5000 g/mol.

According to a preferred embodiment, in formula (1) and particularly in formula (2) R¹ is a substituted or unsubstituted C₁₋₂₀ hydrocarbon residue, Z is a covalent bond and X¹ is a nitrogen atom. It is even more preferred that in formula (1) R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, Z is a covalent bond and X¹ is a nitrogen atom. Most preferably, in formula (1) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group, Z is a covalent bond and X¹ is a nitrogen atom.

Alternatively, it is preferred that in formula (1) and particularly in formula (2) R¹ is a polymer having a molecular weight of 150 to 500000 g/mol, preferably from 500 to 5000 g/mol, Z is a covalent bond and X¹ is a nitrogen atom. It is also preferred that in formula (1) and particularly in formula (2) R¹ is a synthetic polymer, Z is a covalent bond and X¹ is a nitrogen atom. It is most preferred that in formula (1) and particularly in formula (2) R¹ is a synthetic polymer having a molecular weight of 150 to 500000 g/mol, preferably from 500 to 5000 g/molZ is a covalent bond and X¹ is a nitrogen atom.

In accordance with a preferred embodiment, the alkylating agent according to formula (1) or (2) is linked to the surface of a support either covalently through a linker, electrostatically, or by adsorption. It is particularly preferred that the alkylating agent is covalently linked to the surface of the support. Even more preferably, the support is a synthetic polymer, a biopolymer, a metal, silicate, or a ceramic. It is particularly preferred that the method is performed in a tube reactor filled with the alkylating agent linked to the support. This allows to simplify the generation oif the alkylating as well as the separation of the alkylating agent and the alkylated substrate A supported compound can be easily separated from the reaction mixture, thus increasing the purity of the final product with less effort. The supported compounds can be furthermore regenerated by treatment with an alkylating agent. Moreover, it allows for the integration in to other systems such as flow and batch.

Preferably, in formulae (1) and (2) A⁻ is an anion which is not alkylated to a degree of more than 10% when being in contact with methyl iodide for 10 minutes at room temperature, at atmospheric pressure in a molar ratio of A⁻ to methyl iodide of 1:1. This degree is a parameter specifying that the nucleophilicity of the anion A⁻ is less than that of methyl iodide. It is even more preferred that in formulae (1) and (2) A⁻ is an anion containing at least one of a fluorinated hydrocarbon residue, a perfluorinated hydrocarbon residue, and/or an anionic charge centred about a borate, phosphate, phosphonate, sulfate, sulfonate, acetate, perchlorate, (bi)carbonate, triazolate, and/or cyanamide structure. Even more preferably, in formulae (1) and (2) the anion A⁻ is selected from the group consisting of BR¹⁴ ₄⁻, PF₆⁻, B(C₆H₅)₄⁻, triflate, TFSI, FSI, BETI, TFSAM, TSAC and DCA, wherein in BR¹⁴₄⁻ R¹⁴ is selected from the group consisiting of substituted or unsubstituted C₁₋₂₀ hydrocarbon residues and halogen atoms. Most preferably, the anion is selected from the group consisting of TFSI, BR¹⁴ ₄⁻, PF₆⁻ and B(C₆H₅)₄⁻, wherein in BR¹⁴₄⁻ R¹⁴ is selected from the group consisiting of substituted or unsubstituted C₁₋₁₀ hydrocarbon residues and halogen atoms. Since these anions cannot be alkylated further by methyl iodide, they do not form volatile compounds during storage or reaction and also improve the thermal stability of such alkylating agents compared with anions that may be alkylated by methyl iodide. Thus, the above mentioned anions improve the security of such alkylating agents for both, storage and handling.

In accordance with yet another preferred embodiment, the alkylation agent is an ionic liquid, more preferably a room temperature ionic liquidAs a room-temperature ionic liquid, the alkylation agent of this embodiment is non-volatile and thus safe. Yet, it can be dissolved easily in another solvent or used as a solvent itself. It can also be injected, sprayed, painted and coated with or without solvent addition. It is also preferred to synthesise an ionic liquid, preferably a room temperature ionic liquid using the method according to the invention.

It is preferable, that in the method an iodide containing salt is added to the alkylating agent in accordance with formula (1) or (2), i.e. the alkylating agent is reacted with the molecule or ion in the presence of an iodide containing salt. The molar ratio of the alkylating agent to the iodide containing salt is preferably in a range of 0.01 to 100.000, more preferably in a range of 0.1 to 10.000, even more preferably in a range of 0.1 to 100. When the iodide containing salt is added, the reactivity of the alkylating agent is increased, while the security aspects of the alkylating agent duing storage are not negatively influenced. Any volatile compound resulting from an alkylation of the iodide is readily consumed in a subsequent reaction with the molecule or the ion to be alkylated, and thus the security for storage and handling is not negatively influenced in a significant manner.

According to another preferred embodiment, the alkylation is performed at a temperature in the range of -50 to 200 °C, more preferably at a temperature in the range of -30 to 100 °C and even more preferably at a temperature in the range of 10 to 100 °C. If the method for alkylation is performed below -50 °C, the reaction rate of the alkylation reaction becoms to slow. At temperatures above 200 °C there is a risk that side reaction appear more frequently and the alkylating agent decomposes before the molecule or ion is alkylated in a desired amount.

Different solvents may be used in the method for alkylating a molecule or ion, i.e. the alkylating agent is reacted with the molecule or ion in the presence of a solvent. It is preferred that at least one of water, N,N-dimethylformamide, dimethyl sulfoxide, N,N-dimethylacetamide, acetonitrile and/or acetone is used as a solvent.

According to another preferred embodiment, an organic molecule or a metalorganic molecule is alkylated. It is further preferred that a molecule is alkylated which comprises at least one of a nitrogen atom, a sulphur atom, an oxygen atom and/or a phosphorus atom. It is particularly preferred that a molecule is alkylated which comprises at least one of a primary amine, a secondary amine, a tertiary amine, an ester, an aldehyde, a ketone, an amide, a carboxylate group, an azine group, a phosphin group, a borate group and/or a nitrogen atom in an aromatic system. It is preferable that a biomolecule is alkylated, which has preferably a molecular weight of equal to or greater than 75 g/mol, more preferably of greater than 112 g/mol.

### Second particular preferred embodiment of the method of the present invention

According to a second aspect of the present invention, the alkylating agent is preferably a compound according to formula (3): 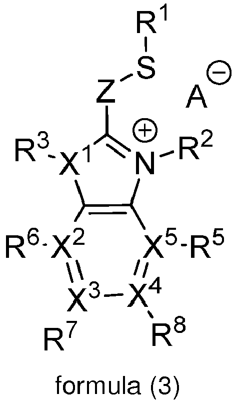 wherein in formula (3)
R¹ is a substituted or unsubstituted C₁₋₂₀ hydrocarbon residue or a polymer, R² is a substituted or unsubstituted C₁₋₂₀ hydrocarbon residue or a hydrogen atom, R³ is absent, a substituted or unsubstituted C₁₋₂₀ hydrocarbon residue or a hydrogen atom, R⁶, R⁷, R⁸ and R⁹ are independently from each other selected from the group consisting of a hydrogen atom, an oxygen atom, a sulfur atom, substituted or unsubstituted C₁₋₂₀ hydrocarbon residues and electron withdrawing groups, and/or any of R⁶, R⁷, R⁸ and R⁹ is linked to another one of R⁶, R⁷, R⁸ and R⁹ to form a cyclic structure, X¹ is an oxygen atom, a nitrogen atom or a sulfur atom, wherein R³ is not present in formula (3) if X¹ is an oxygen atom or a sulfur atom, X², X³, X⁴ and X⁵ are independently from each other selected from the group consisting of a carbon atom, a nitrogen atom, an oxygen atom and a sulfur atom, and/or X², X³, X⁴ and X⁵ form an aromatic ring and A⁻ is an anion. Due to the aromatic ring formed by X², X³, X⁴ and X⁵, the compound according to formula (3) is provided with a large aromatic system and it was found that thereby the reactivity for alkylating a molecule or an ion is increased. The compound according to the formula (3) has more points to introduce functionality, such as attachment to a solid-support. Moreover, it further tunes the already heightened reactivity of the system.

It is preferred that in formula (3) X¹ is a nitrogen atom. It is also preferred that in formula (3) X², X³, X⁴ and X⁵ are carbon atoms. It is in partucularly preferred that in formula (3) X¹ is a nitrogen atom and X², X³, X⁴ and X⁵ are carbon atoms. Residues X² to X⁵ are preferably a combination of either carbon, nitrogen or oxygen. The caffeine structure has the highest reactivity and has nitrogen and carbon. More electronegative elements increase reactivity as long as aromaticity is retained.

According to a further embodiment, in formula (3) R² and R³ are preferably independently from each other a substituted or unsubstituted C₁₋₂₀ hydrocarbon residue. It is particularly preferred that in formula (3) R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. More preferably, in formula (3) R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group. Most preferably, in formula (3) R² and R³ are independently from each other a substituted or unsubstituted methyl group, ethyl group, phenyl group, or benzyl group. The reactivity of the compound increases, from least reactive to most reactive, from alkyl to benzyl to alkenyl to phenyl.

In a preferred embodiment, in formula (3) R² and R³ are independently from each other a substituted or unsubstituted C₁₋₂₀ hydrocarbon residue, X¹ is a nitrogen atom and X², X³, X⁴ and X⁵ are carbon atoms. It is particularly preferred that in formula (3) R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, X¹ is a nitrogen atom and X², X³, X⁴ and X⁵ are carbon atoms. It is even more preferred that in formula (3) R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group, X¹ is a nitrogen atom and X², X³, X⁴ and X⁵ are carbon atoms. Most preferably, in formula (3) R² and R³ are independently from each other a substituted or unsubstituted methyl group, ethyl group, phenyl group, or benzyl group, X¹ is a nitrogen atom and X², X³, X⁴ and X⁵ are carbon atoms.

According to another preferred embodiment, in formula (3) R² is equal to R³. This facilitates the synthesis of the compound. It is further preferred that in formula (3) R² is equal to R³, X¹ is a nitrogen atom and X², X³, X⁴ and X⁵ are carbon atoms. It is also preferred that in formula (3) R² is equal to R³ and R² and R³ are selected from the group consisting of a substituted or unsubstituted C₁₋₂₀ hydrocarbon residue. It is particularly preferred that in formula (3) R² is equal to R³ and R² and R³ are selected from the group consisting of a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. Even more preferrably in formula (3) R² is equal to R³ and R² and R³ are selected from the group consisting of a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group. Most preferably, in formula (3) R² is equal to R³ and R² and R³ are selected from the group consisting of a substituted or unsubstituted methyl group, ethyl group or benzyl group.

According to another preferred embodiment, in formula (3) R² is equal to R³, R² and R³ are selected from the group consisting of a substituted or unsubstituted C₁₋₂₀ hydrocarbon residue X¹ is a nitrogen atom and X², X³, X⁴ and X⁵ are carbon atoms. It is further preferred that in formula (3) R² is equal to R³, R² and R³ are selected from the group consisting of a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, X¹ is a nitrogen atom and X², X³, X⁴ and X⁵ are carbon atoms. It is particularly preferred that in formula (3) R² is equal to R³, R² and R³ are selected from the group consisting of a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group, X¹ is a nitrogen atom and X², X³, X⁴ and X⁵ are carbon atoms. Even more preferrably, in formula (3) R² is equal to R³, R² and R³ are selected from the group consisting of a substituted or unsubstituted methyl group, ethyl group or benzyl group, X¹ is a nitrogen atom and X², X³, X⁴ and X⁵ are carbon atoms.

In formula (3) R¹ is preferably a substituted or unsubstituted C₁₋₂₀ hydrocarbon residue. It is even more preferred that in formula (3) R¹ contains at least one of a hydroxy group and/or an amine group. Some examples for R¹ in formula (3) are a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. It is particularly preferred that in formula (3) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group. Even more preferably, in formula (3) R¹ is a substituted or unsubstituted C₁₋₆ alkyl group, a substituted or unsubstituted C₄₋₈ cycloalkyl group, a substituted or unsubstituted C₂₋₆ alkenyl group or a substituted or unsubstituted C₂₋₆ alkinyl group. Most preferably, in formula (3) R¹ is a methyl group or an ethly group. Alternatively, residue R¹ may be a hydrocarbon residue being attached to a solid-support. This acts as a means to attach the solid-support to a nucleophile.

Alternatively, R¹ is a polymer having a molecular weight of 150 to 500000 g/mol, preferably from 500 to 5000 g/mol. It is particularly preferred that R¹ is a synthetic polymer and even more preferred that R¹ is a synthetic polymer having a molecular weight of 150 to 500000 g/mol, preferably from 500 to 5000 g/mol.

According to another preferred embodiment, in formula (3) R¹ is a substituted or unsubstituted C₁₋₂₀ hydrocarbon residue, X¹ is a nitrogen atom and X², X³, X⁴ and X⁵ are carbon atoms. It is further preferred that in formula (3) R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, X¹ is a nitrogen atom and X², X³, X⁴ and X⁵ are carbon atoms. It is particularly preferred that in formula (3) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group, X¹ is a nitrogen atom and X², X³, X⁴ and X⁵ are carbon atoms.

According to still another preferred embodiment, in formula (3) R¹ is a polymer having a molecular weight of 150 to 500000 g/mol, preferably from 500 to 5000 g/mol, X¹ is a nitrogen atom and X², X³, X⁴ and X⁵ are carbon atoms. It is further preferred that in formula (3) R¹ is a synthetic polymer, X¹ is a nitrogen atom and X², X³, X⁴ and X⁵ are carbon atoms. It is particularly preferred that in formula (3) R¹ is a synthetic polymer having a molecular weight of 150 to 500000 g/mol, preferably from 500 to 5000 g/mol, X¹ is a nitrogen atom and X², X³, X⁴ and X⁵ are carbon atoms.

In accordance with a further preferred embodiment of the present invention, in formula (3) at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups. As a consequence of providing at least one such electron withdrawing group, the bond between the sulfur atom and R¹ is weakened and the reactivity of the compound according to forumla (3) is increased. More preferably, in formula (3) at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups and X¹ is a nitrogen atom. Even more preferably, in formula (3) at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms.

In another preferred embodiment, in formula (3) R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. It is further preferred that in formula (3) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group, at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. Alternatively, in formula (3) R¹ is a polymer having a molecular weight of 150 to 500000 g/mol, preferably from 500 to 5000 g/mol, at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. It is further preferred that in formula (3) R¹ is a synthetic polymer, at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. Even more preferably, in formula (3) R¹ is a synthetic polymer having a molecular weight of 150 to 500000 g/mol, preferably from 500 to 5000 g/mol, at least one of R⁶, R⁷, R⁸ and R⁹ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms.

According to another preferred embodiment of the second aspect of the present invention, in formula (3) at least one of R⁶, R⁷, R⁸ and R⁹ is linked to another one of R⁶, R⁷, R⁸ and R⁹ to form a cyclic structure which preferably is aromatic. Such a linking to an aromatic structure will increase reactivity. Anthracene for example produces a highly reactive alkylation agent.

In accordance with a preferred embodiment, the alkylating agent according to formula (3) is linked to the surface of a support either covalently through a linker, electrostatically, or by adsorption. It is particularly preferred that the alkylating agent is covalently linked to the surface of the support. Even more preferably, the support is a synthetic polymer, a biopolymer, a metal, silicate, or a ceramic. It is particularly preferred that the method is performed in a tube reactor filled with the alkylating agent linked to the support. Such supported compounds can be separated from the reaction mixture, thus increasing the purity of the final product with less effort. Furthermore, the supported compounds can be regenerated by treatment with alkylating agent. This allows for integration in to other systems such as flow and batch.

Preferably, in formula (3) A⁻ is an anion which is not alkylated to a degree of more than 10% when being in contact with methyl iodide for 10 minutes at room temperature, at atmospheric pressure in a molar ratio of A⁻ to methyl iodide of 1:1. It is even more preferred that A⁻ is an anion containing at least one of a fluorinated hydrocarbon residue, a perfluorinated hydrocarbon residue, and/or an anionic charge centred about a borate, phosphate, phosphonate, sulfate, sulfonate, acetate, (bi)carbonate, perchlorate, triazolate, and/or cyanamide structure. Even more preferably, the anion is selected from the group consisting of BR¹⁴₄⁻, PF₆⁻ , B(C₆H₅)₄⁻, triflate, TFSI, FSI, BETI, TFSAM, TSAC and DCA, wherein in BR¹⁴₄⁻ R¹⁴ is selected from the group consisiting of substituted or unsubstituted C₁₋₂₀ hydrocarbon residues and halogen atoms. Most preferably, the anion is selected from the group consisting of TFSI, BR¹⁴ ₄⁻, PF₆⁻ and B(C₆H₅)₄⁻, wherein in BR¹⁴₄⁻R¹⁴ is selected from the group consisiting of substituted or unsubstituted C₁₋₁₀ hydrocarbon residues and halogen atoms. Since these anions cannot be alkylated further by methyl iodide, they do not form volatile compounds during storage or reaction and also improve the termal stability of such alkylating agents compared with anions that may be alkylated by methyl iodide. Thus, the above mentioned anions improve the security of such alkylating agents for both, storage and handling.

In accordance with another preferred embodiment, the alkylation agent in accordance with formula (3) is an ionic liquid, more preferably a room temperature ionic liquid. As a room-temperature ionic liquid, the alkylation agent is non-volatile and thus safe. Yet, it can be dissolved easily in another solvent or used as a solvent itself. It can also be injected, sprayed, painted or coated with or without solvent addition. It is also preferred to synthesise an ionic liquid, preferably a room temperature ionic liquid using the method according to the invention.

It is preferable, that in the method an iodide containing salt is added to the alkylating agent in accordance with formula (3). The molar ratio of the alkylating agent to the iodide containing salt is preferably in a range of 0.01 to 100.000, more preferably in a range of 0.1 to 10.000, even more preferably in a range of 0.1 to 100. When the iodide containing salt is added, the reactivity of the alkylating agent is increased. When the iodide containing salt is added, the reactivity of the alkylating agent is increased, while the security aspects of the alkylating agent duing storage are not negatively influenced. Any volatile compound resulting from an alkylation of the iodide is readily consumed in a subsequent reaction with the molecule or the ion to be alkylated, and thus the security for storage and handling is not negatively influenced in a significant manner.

According to another preferred embodiment, the alkylation is performed at a temperature in the range of -50 to 200 °C, more preferably at a temperature in the range of -30 to 100 °C and even more preferably at a temperature in the range of 10 to 100 °C. If the method for alkylation is performed below -50 °C, the reaction rate of the alkylation reaction becoms to slow. At temperatures above 200 °C there is a risk that side reaction appear more frequently and the alkylating agent decomposes before the molecule or ion is alkylated in a desired amount.

Different solvents may be used in the method for alkylating a molecule or ion. It is preferred that at least one of water, N,N-dimethylformamide, dimethyl sulfoxide, N,N-dimethylacetamide, acetonitrile and/or acetone is used as a solvent.

According to another preferred embodiment, an organic molecule or a metalorganic molecule is alkylated. It is further preferred that a molecule is alkylated which comprises at least one of a nitrogen atom, a sulphur atom, an oxygen atom and/or a phosphorus atom. It is particularly preferred that a molecule is alkylated which comprises at least one of a primary amine, a secondary amine, a tertiary amine, an ester, an aldehyde, a ketone, an amide, a carboxylate group, an azine group, a phosphin group, a borate group and/or a nitrogen atom in an aromatic system. It is preferable that a biomolecule is alkylated, which has preferably a molecular weight of equal to or greater than 75 g/mol, more preferably of greater than 112 g/mol.

### Third particular preferred embodiment of the method of the present invention

According to a third aspect of the present invention, the alkylating agent is preferably a compound according to formula (4): 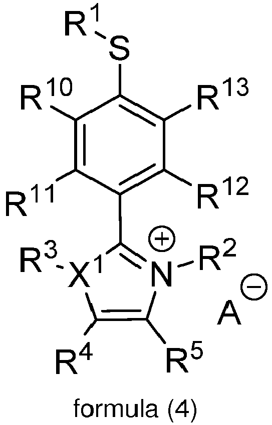 wherein in formula (4) R¹ is a substituted or unsubstituted C₁₋₂₀ hydrocarbon residue or a polymer, R² is a substituted or unsubstituted C₁₋₂₀ hydrocarbon residue or a hydrogen atom, R³ is absent, a substituted or unsubstituted C₁₋₂₀ hydrocarbon residue or a hydrogen atom, R⁴ and R⁵ are linked with each other to form an aromatic group or are independently from each other selected from the group consisting of a hydrogen atom, substituted and unsubstituted C₁₋₂₀ hydrocarbon residues and electron withdrawing groups, R¹⁰ to R¹³ are independently selected from the group consisting of hydrogen, substituted and unsubstituted C₁₋₂₀ hydrocarbon residues and electron withdrawing groups, X¹ is an oxygen atom, a nitrogen atom or an sulfur atom, wherein R³ is not present in formula (4) if X¹ is an oxygen atom or a sulfur atom, and A⁻ is an anion. Compounds according to the formula (4) may be easily synthesized by methods that can be facilitated in one step. The have more positions for the manipulation of the structure - such as for changing the reactivity, adding functionality or attachment to solid support.
According to a preferred embodiment, in formula (4) R¹⁰ to R¹³ are hydrogen atoms. It is also preferred that in formula (4) X¹ is a nitrogen atom. It is even more preferred that in formula (4) R¹⁰ to R¹³ are hydrogen atoms and X¹ is a nitrogen atom.

According to another preferred embodiment, in formula (4) R⁴ and R⁵ are hydrogen atoms. It is even more preferred that in formula (4) R⁴ and R⁵ are hydrogen atoms and that R¹⁰ to R¹³ are hydrogen atoms.

In accordance with a further preferred embodiment, in formula (4) R⁴ and R⁵ are hydrogen atoms and X¹ is a nitrogen atom. With this respect, it is particularly preferred that in formula (4) R⁴ and R⁵ are hydrogen atoms, R¹⁰ to R¹³ are hydrogen atoms and X¹ is a nitrogen atom.

According to a further embodiment, in formula (4) R² and R³ are preferably independently from each other a substituted or unsubstituted C₁₋₂₀ hydrocarbon residue. It is particularly preferred that in formula (4) R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. More preferably, in formula (4) R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group. Most preferably, in formula (4) R² and R³ are independently from each other a substituted or unsubstituted methyl group, ethyl group or benzyl group.

In a preferred embodiment, in formula (4) R² and R³ are independently from each other a substituted or unsubstituted C₁₋₂₀ hydrocarbon residue, X¹ is a nitrogen atom and X², X³, X⁴ and X⁵ are carbon atoms. It is particularly preferred that in formula (4) R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, X¹ is a nitrogen atom and X², X³, X⁴ and X⁵ are carbon atoms. It is even more preferred that in formula (4) R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group, X¹ is a nitrogen atom and X², X³, X⁴ and X⁵ are carbon atoms. Most preferably, in formula (4) R² and R³ are independently from each other a substituted or unsubstituted methyl group, ethyl group, phenyl group, or benzyl group, X¹ is a nitrogen atom and X², X³, X⁴ and X⁵ are carbon atoms.

In accordance with another preferred embodiment, in formula (4) R² is equal to R³. It is further preferred that in formula (4) R² is equal to R³ and X², X³, X⁴ and X⁵ are carbon atoms. It is also preferred that in formula (4) R² is equal to R³ and R² and R³ are selected from the group consisting of a substituted or unsubstituted C₁₋₂₀ hydrocarbon residue. It is particularly preferred that in formula (4) R² is equal to R³ and R² and R³ are selected from the group consisting of a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. Even more preferrably, in formula (4) R² is equal to R³ and R² and R³ are selected from the group consisting of a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group. Most preferably, in formula (4) R² is equal to R³ and R² and R³ are selected from the group consisting of a substituted or unsubstituted methyl group, ethyl group, phenyl group, or benzyl group.

In another preferred embodiment in formula (4) R² is equal to R³, R² and R³ are selected from the group consisting of a substituted or unsubstituted C₁₋₂₀ hydrocarbon residue X¹ is a nitrogen atom and X², X³, X⁴ and X⁵ are carbon atoms.lt is further preferred that in formula (4) R² is equal to R³, R² and R³ are selected from the group consisting of a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, X¹ is a nitrogen atom and X², X³, X⁴ and X⁵ are carbon atoms. It is particularly preferred that in formula (4) R² is equal to R³, R² and R³ are selected from the group consisting of a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group, X¹ is a nitrogen atom and X², X³, X⁴ and X⁵ are carbon atoms. Most preferrably, in formula (4) R² is equal to R³, R² and R³ are selected from the group consisting of a substituted or unsubstituted methyl group, ethyl group, phenyl group, or benzyl group, X¹ is a nitrogen atom and X², X³, X⁴ and X⁵ are carbon atoms.

In formula (4) R¹ is preferably a substituted or unsubstituted C₁₋₂₀ hydrocarbon residue. It is even more preferred that in formula (4) R¹ contains at least one of a hydroxy group and/or an amine group. Some examples for R¹ in formula (4) are a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. It is particularly preferred that in formula (4) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group. Even more preferably, in formula (4) R¹ is a substituted or unsubstituted C₁₋₆ alkyl group, a substituted or unsubstituted C₄₋₈ cycloalkyl group, a substituted or unsubstituted C₂₋₆ alkenyl group or a substituted or unsubstituted C₂₋₆ alkinyl group. Most preferably, in formula (4) R¹ is a methyl group or an ethly group.

Alternatively, in formula (4) R¹ is a polymer having a molecular weight of 150 to 500000 g/mol, preferably from 500 to 5000 g/mol. It is particularly preferred that R¹ is a synthetic polymer and even more preferred that R¹ is a synthetic polymer having a molecular weight of 150 to 500000 g/mol, preferably from 500 to 5000 g/mol.

According to another preferred embodiment, in formula (4) R¹ is a substituted or unsubstituted C₁₋₂₀ hydrocarbon residue, R⁴ and R⁵ are hydrogen atoms and X¹ is a nitrogen atom. It is further preferred that in formula (4) R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, R⁴ and R⁵ are hydrogen atoms and X¹ is a nitrogen atom. It is particularly preferred that in formula (4) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group, R⁴ and R⁵ are hydrogen atoms and X¹ is a nitrogen atom.

In accordance with an alternative preferred embodiment, in formula (4) R¹ is a polymer having a molecular weight of 150 to 500000 g/mol, preferably from 500 to 5000 g/mol, R⁴ and R⁵ are hydrogen atoms and X¹ is a nitrogen atom. It is further preferred that in formula (4) R¹ is a synthetic polymer, R⁴ and R⁵ are hydrogen atoms and X¹ is a nitrogen atom. It is particularly preferred that in formula (4) R¹ is a synthetic polymer having a molecular weight of 150 to 500000 g/mol, preferably from 500 to 5000 g/mol, R⁴ and R⁵ are hydrogen atoms and X¹ is a nitrogen atom.

It is preferred that in formula (4) at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups. As a consequence of providing such electron withdrawing groups, the bond between the sulfur atom and R¹ is weakened and the reactivity of the compound according to forumla (4) as an alkylating agent is increased. It is further preferred that in formula (4) at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups and X¹ is a nitrogen atom. In another preferred embodiment, in formula (4) R⁴ and R⁵ are hydrogen atoms, at least one of R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. According to another preferred embodiment, in formula (4) R¹⁰, R¹¹, R¹², and R¹³ are hydrogen atoms, at least one of R⁴ and R⁵ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms.

It is also preferred that in formula (4) R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. It is even further preferred that in formula (4) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group, R⁴ and R⁵ are hydrogen atoms, at least one of R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms.

In accordance with yet another preferred embodiment, in formula (4) R¹ is a polymer having a molecular weight of 150 to 500000 g/mol, preferably from 500 to 5000 g/mol, at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. It is also preferred that in formula (4) R¹ is a synthetic polymer, at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms. Most preferably, in formula (4) R¹ is a synthetic polymer having a molecular weight of 150 to 500000 g/mol, preferably from 500 to 5000 g/mol, at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups, X¹ is a nitrogen atom and X², X³, X⁴ and X⁵ are carbon atoms.

In accordance with a preferred embodiment, the compound according to formula (4) is linked to the surface of a support either covalently through a linker, electrostatically, or by adsorption. It is particularly preferred that the alkylating agent is covalently linked to the surface of the support. Even more preferably, the support is a synthetic polymer, a biopolymer, a metal, silicate, or a ceramic. Such supported compounds can be separated from the reaction mixture, thus increasing the purity of the final product with less effort. Furthermore, the supported compounds can be regenerated by treatment with alkylating agent. This allows for integration in to other systems such as flow and batch. It is particularly preferred that the method is performed in a tube reactor filled with the alkylating agent linked to the support.

Preferably, in formula (4) A⁻ is an anion containing at least one of a fluorinated hydrocarbon residue, a perfluorinated hydrocarbon residue, and/or an anionic charge centred about a borate, phosphate, phosphonate, sulfate, sulfonate, acetate, perchlorate, triazolate, and/or cyanamide structure. Even more preferably, the anion is selected from the group consisting of BR¹⁴₄⁻, PF₆⁻ , B(C₆H₅)₄⁻, triflate, TFSI, FSI, BETI, TFSAM, TSAC and DCA, wherein in BR¹⁴₄⁻ R¹⁴ is selected from the group consisiting of substituted or unsubstituted C₁₋₂₀ hydrocarbon residues and halogen atoms. Most preferably, the anion is selected from the group consisting of TFSI, BR¹⁴₄⁻, PF₆⁻ and B(C₆H₅)₄⁻, wherein in BR¹⁴₄⁻ R¹⁴ is selected from the group consisiting of substituted or unsubstituted C₁₋₁₀ hydrocarbon residues and halogen atoms. Since these anions cannot be alkylated further by methyl iodide, they do not form volatile compounds during storage or reaction and also improve the termal stability of such compounds compared with anions that may be alkylated by methyl iodide. Thus, the above mentioned anions improve the security of such alkylating agents for both, storage and handling.

In accordance with another preferred embodiment, the alkylation agent is an ionic liquid, more preferably a room temperature ionic liquid. As a room-temperature ionic liquid, the alkylation agent is non-volatile and thus safe. Yet, it can be dissolved easily in another solvent or used as a solvent itself. It can also be injected, sprayed, painted or coated with or without solvent addition. It is also preferred to synthesise an ionic liquid, preferably a room temperature ionic liquid using the method according to the invention.

It is preferable, that in the method an iodide containing salt is added to the alkylating agent according to formula (4). The molar ratio of the alkylating agent according to formula (4) to the iodide containing salt is preferably in a range of 0.01 to 100.000, more preferably in a range of 0.1 to 10.000, even more preferably in a range of 0.1 to 100. When the iodide containing salt is added, the reactivity of the alkylating agent is increased. When the iodide containing salt is added, the reactivity of the alkylating agent is increased, while the security aspects of the alkylating agent duing storage are not negatively influenced. Any volatile compound resulting from an alkylation of the iodide is readily consumed in a subsequent reaction with the molecule or the ion to be alkylated, and thus the security for storage and handling is not negatively influenced in a significant manner.

According to yet another preferred embodiment, the alkylation is performed at a temperature in the range of -50 to 200 °C, more preferably at a temperature in the range of -30 to 100 °C and even more preferably at a temperature in the range of 10 to 100 °C. If the method for alkylation is performed below -50 °C, the reaction rate of the alkylation reaction becoms to slow. At temperatures above 200 °C there is a risk that side reaction appear more frequently and the alkylating agent decomposes before the molecule or ion is alkylated in a desired amount.

Different solvents may be used in the method for alkylating a molecule or ion. It is preferred that at least one of water, N,N-dimethylformamide, dimethyl sulfoxide, N,N-dimethylacetamide, acetonitrile and/or acetone is used as a solvent.

According to another preferred embodiment, an organic molecule or a metalorganic molecule is alkylated. It is further preferred that a molecule is alkylated which comprises at least one of a nitrogen atom, a sulphur atom, an oxygen atom and/or a phosphorus atom. It is particularly preferred that a molecule is alkylated which comprises at least one of a primary amine, a secondary amine, a tertiary amine, an ester, an aldehyde, a ketone, an amide, a carboxylate group, an azine group, a phosphin group, a borate group and/or a nitrogen atom in an aromatic system. It is preferable that a biomolecule is alkylated, which has preferably a molecular weight of equal to or greater than 75 g/mol, more preferably of greater than 112 g/mol.

Another aspeict of the present invention is a compound according to formula (1a): 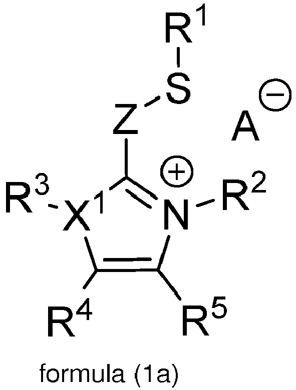 wherein in formula (1a) R¹ is a substituted or unsubstituted C₁₋₂₀ hydrocarbon residue or a polymer, R² is a substituted or unsubstituted C₁₋₂₀ hydrocarbon residue or a hydrogen atom, R³ is absent, a substituted or unsubstituted C₁₋₂₀ hydrocarbon residue or a hydrogen atom, R⁴ and R⁵ are linked with each other to form an aromatic group or are independently from each other selected from the group consisting of a hydrogen atom, substituted and unsubstituted C₁₋₂₀ hydrocarbon residues and electron withdrawing groups, X¹ is an oxygen atom, a nitrogen atom or a sulfur atom, wherin R³ is not present in formula (1a) if X¹ is an oxygen atom or a sulfur atom, Z is a covalent bond or an aromatic group and A⁻ is an anion which is not alkylated to a degree of more than 10% when being in contact with methyl iodide for 10 minutes at room temperature, at atmospheric pressure in a molar ratio of A⁻ to methyl iodide of 1:1.

Since the anion A⁻ is not easily alkylated, the formation of a volatile compound during storage of the compound according to formula (1a) does not occur, providing for safe storage and handling of the compound according to forumla (1a), while still providing a powerful alkylation agent.

It is preferable that in the compound according to formula (1a) A⁻ is an anion containing at least one of a fluorinated hydrocarbon residue, a perfluorinated hydrocarbon residue, and/or an anionic charge centred about a borate, phosphate, phosphonate, sulfate, sulfonate, acetate, perchlorate, triazolate, (bi)carbonate, and/or cyanamide structure. More preferably, the anion in formula (1a) is selected from the group consisting of BR¹⁴₄⁻, PF₆⁻, B(C₆H₅)₄⁻, triflate, TFSI, FSI, BETI, TFSAM, TSAC and DCA, wherein in BR¹⁴₄⁻ R¹⁴ is a moiety selected from the group consisiting of substituted or unsubstituted C₁₋₂₀ hydrocarbon residues and halogen atoms. Most preferably, the anion is selected from the group consisting of TFSI, BR¹⁴ ₄⁻, PF₆⁻ and B(C₆H₅)₄, wherein in BR¹⁴₄⁻ R¹⁴ is selected from the group consisiting of substituted or unsubstituted C₁₋₁₀ hydrocarbon residues and halogen atoms.

In accordance with another preferred embodiment, the compound according to formula (1a) is an ionic liquid, more preferably a room temperature ionic liquid. As a room-temperature ionic liquid, the alkylation agent is non-volatile and thus safe. Yet, it can be dissolved easily in another solvent or used as a solvent itself. It can also be injected, sprayed, painted or coated with or without solvent addition.

According to another preferred embodiment, the compound according to formula (1a) is linked to a surface of a support either covalently through a linker, electrostatically, or by adsorption.

It is preferable that in formula (1a) R⁴ and R⁵ are hydrogen atoms. According to another preferred embodiment of formula (1a), Z is a covalent bond. In accordance with yet another preferred embodiment, in formula (1a) X¹ is a nitrogen atom.

It is further preferable that in formula (1a) R⁴ and R⁵ are hydrogen atoms and Z is a covalent bond and even further preferable that R⁴ and R⁵ are hydrogen atoms, Z is a covalent bond and X¹ is a nitrogen atom.

Preferably, in formula (1a) R² and R³ are independently from each other a substituted or unsubstituted C₁₋₂₀ hydrocarbon residue. It is further preferred that in formula (1a) R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. Even more preferably, in formula (1a) R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group. Most preferably, in formula (1a) R² and R³ are independently from each other a substituted or unsubstituted methyl group, ethyl group or benzyl group.

In accordance with a further preferred embodiment, in formula (1a) R² and R³ are independently from each other a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, R⁴ and R⁵ are hydrogen atoms, Z is a covalent bond and X¹ is a nitrogen atom. It is even more preferred that in formula (1a) R² and R³ are independently from each other a substituted or unsubstituted C₁₋₁₀ alkyl group, a substituted or unsubstituted C₃₋₁₀ cycloalkyl group, a substituted or unsubstituted C₂₋₁₀ alkenyl group or a substituted or unsubstituted C₂₋₁₀ alkinyl group, R⁴ and R⁵ are hydrogen atoms, Z is a covalent bond and X¹ is a nitrogen atom. Most preferably, in formula (1a) R² and R³ are independently from each other a substituted or unsubstituted methyl group, ethyl group, phenyl group, or benzyl group, R⁴ and R⁵ are hydrogen atoms, Z is a covalent bond and X¹ is a nitrogen atom.

In a further preferred embodiment, in formula (1a) R² is equal to R³. If R² is equal to R³ synthesis of the compound according to formula (1a) is easier. It is particularly preferred that in formula (1a) R² is equal to R³, R⁴ and R⁵ are hydrogen atoms, Z is a covalent bond and X¹ is a nitrogen atom.

In formula (1a) R¹ is preferably a substituted or unsubstituted C₁₋₂₀ hydrocarbon residue. It is even more preferred that in formula (1a) R¹ contains at least one of a hydroxy group and/or an amine group. Some examples for R¹ in formula (1a) are a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group. It is particularly preferred that in formula (1a) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group.

Alternatively, in formula (1a) R¹ is a polymer having a molecular weight of 150 to 500000 g/mol, preferably from 500 to 5000 g/mol. It is particularly preferred that in formula (1a) R¹ is a synthetic polymer and even more preferred that in formula (1a) R¹ is a synthetic polymer having a molecular weight of 150 to 500000 g/mol, preferably from 500 to 5000 g/mol.

According to a preferred embodiment, in formula (1a) R¹ is a substituted or unsubstituted C₁₋₂₀ hydrocarbon residue, R⁴ and R⁵ are hydrogen atoms, Z is a covalent bond and X¹ is a nitrogen atom. It is even more preferred that in formula (1a) R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group, R⁴ and R⁵ are hydrogen atoms, Z is a covalent bond and X¹ is a nitrogen atom. Most preferably, in formula (1a) R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group, R⁴ and R⁵ are hydrogen atoms, Z is a covalent bond and X¹ is a nitrogen atom.

Alternatively, it is preferred that in formula (1a) R¹ is a polymer having a molecular weight of 150 to 500000 g/mol, preferably from 500 to 5000 g/mol, R⁴ and R⁵ are hydrogen atoms, Z is a covalent bond and X¹ is a nitrogen atom. It is also preferred that in formula (1a) R¹ is a synthetic polymer, R⁴ and R⁵ are hydrogen atoms, Z is a covalent bond and X¹ is a nitrogen atom. It is most preferred that in formula (1a) R¹ is a synthetic polymer having a molecular weight of 150 to 500000 g/mol, preferably from 500 to 5000 g/mol, R⁴ and R⁵ are hydrogen atoms, Z is a covalent bond and X¹ is a nitrogen atom.

In a further preferred embodiment, at least one of in formula (1a) R⁴ and R⁵ is an electron withdrawing group which is selected from the group consisting of carbonyl groups, hydroxyl groups, halogen atoms, carboxy groups, cyano groups fluorinated and perfluorinated alkyl groups and Z is a covalent bond. As a consequence, the bond between the sulfur atom and R¹ is weakened and the reactivity of the compound according to forumla (1a) is increased.

In accordance with one particularly preferred embodiment of the compound according to formula (1a), X¹ is a nitrogen atom. It is even further preferred that in formula (1a) at least one of R⁴ and R⁵ is an electron withdrawing group which is selected from the group consisting of carbonyl groups, hydroxyl groups, halogen atoms, carboxy groups, cyano groups fluorinated and perfluorinated alkyl groups and Z is a covalent bond. Most preferably, in formula (1a) at least one of R⁴ and R⁵ is an electron withdrawing group which is selected from the group consisting of carbonyl groups, hydroxyl groups, halogen atoms, carboxy groups, cyano groups fluorinated and perfluorinated alkyl groups, Z is a covalent bond and X¹ is a nitrogen atom.

Subsequently, the present invention is described by means of figures, which do, however, not limit the present patent application, wherein:
- Fig. 1: shows the strucutres of preferred anions of the compounds used in the method according to the present invention.
- Fig. 2a: shows a thermogravimetric analysis of compounds according to formula (2), wherein R¹ is one of methyl, ethyl, n-buthyl, n-hexyl, n-octyl or n-dodecyl, R² and R³ are methyl groups, and the anion is iodide.
- Fig. 2b: shows a thermogravimetric analysis of compounds according to formula (2), wherein R¹ is one of methyl, ethyl, n-buthyl, n-hexyl, n-octyl or n-dodecyl, R² and R³ are methyl groups, and the anion is TFSI.
- Fig. 2c: shows a thermogravimetric analysis of compounds according to formula (2), wherein R¹ is one of methyl, ethyl, n-buthyl, n-hexyl, n-octyl or n-dodecyl, R² is benzyl, R³ is a methyl group, and the anion is iodide.
- Fig. 2d: shows a thermogravimetric analysis of compounds according to formula (2), wherein R¹ is one of methyl, ethyl, n-buthyl, n-hexyl, n-octyl or n-dodecyl, R² is benzyl, R³ is a methyl group, and the anion is TFSI.
- Fig. 3a: shows the structure of sevenm alkylating agents, which were used in examples 1 to 7.
- Fig. 3b: shows the reaction scheme of the alkylation of pyridine with any of the alkylating agents shown in Fig. 3a as performed in examples 1 to 7.
- Fig. 4: shows some examples of compounds that were alkylated using an alkylating agent in accordance with the present invention in examples 8 to 37.
- Fig. 5: shows in general the synthesis of the alkylating agent in accordance with the invention.

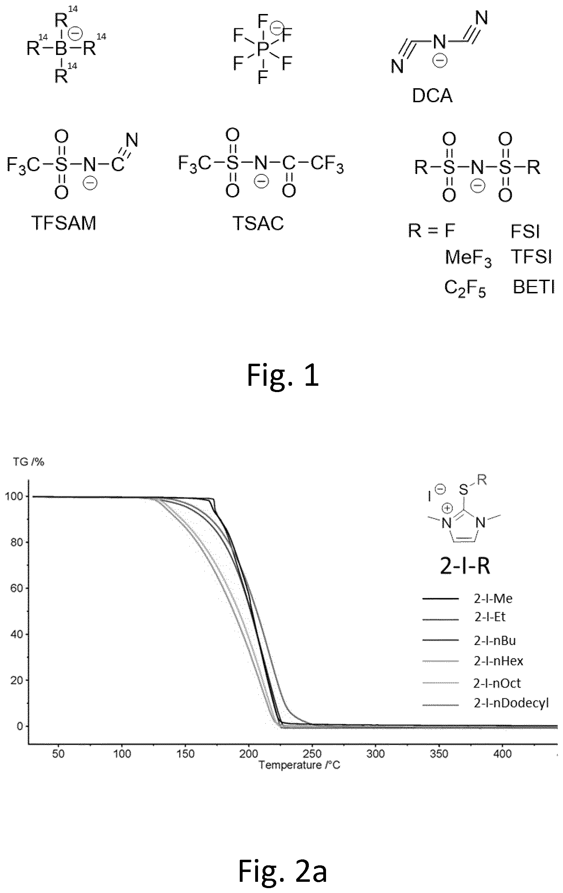

In Fig. 1 several strucutres of preferred anions; as they have been described above for the particular preferred embodiments, are shown. Each of R¹⁴ of the compounds illustrated in Fig. 1 may be a substituted or unsubstituted C₁₋₂₀ hydrocarbon residue or a halogen atom. DCA is the abreviation for dicyanamide, TFSAM for 2,2,2-trifluoromethylsulfonyl-N-cyanoamide, TSAC for 2,2,2-trifluoro-N-(trifluoromethylsulfonyl)acetamide, FSI for bis(fluorosulfonyl)imide, TFSI for bis(trifluoromethanesulfonyl)imide and BETI for bis(perfluoroethylsulfonyl)imide. None of these anions is methylated to a degree of more than 10% when being in contact with methyl iodide for 10 minutes at room temperature, at atmospheric pressure in a molar ratio of the anion to methyl iodide of 1:1.

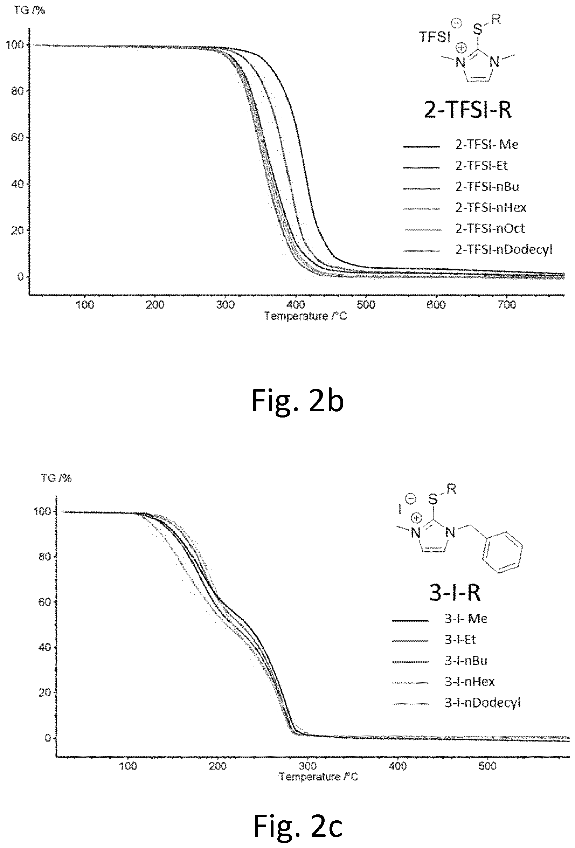

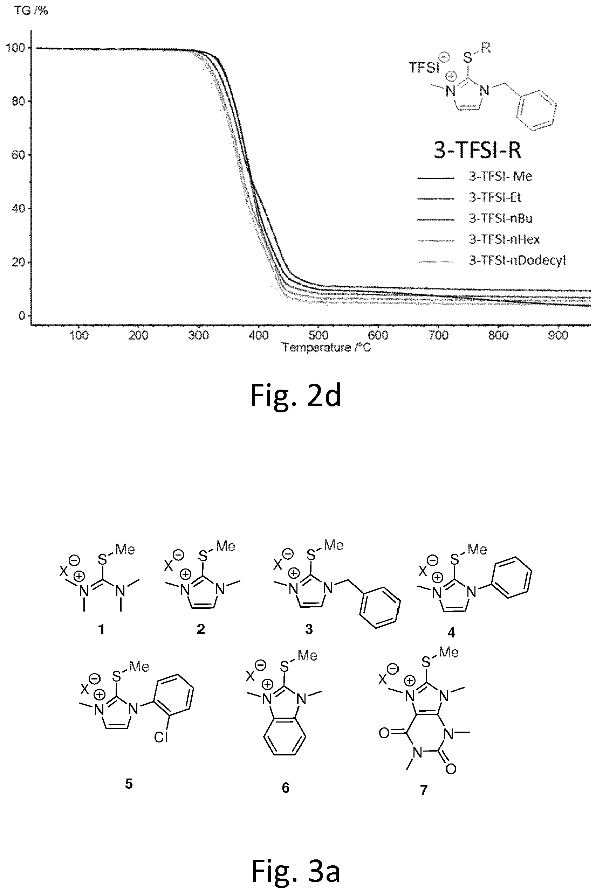

As shown in Fig. 2a - in which a thermogravimetric analysis of compounds according to formula (2), wherein R¹ is one of methyl, ethyl, n-buthyl, n-hexyl, n-octyl or n-dodecyl, R² and R³ are methyl groups, the anion is iodide, is shown - respective compounds according to formula (2), wherein R¹ is one of methyl, ethyl, n-buthyl, n-hexyl, n-octyl or n-dodecyl, R² and R³ are methyl groups, the anion is iodide show a thermal decomposition at around 120°C to 170°C. By changing the anion from iodide to TFSI, as shown in Fig. 2b the thermal compositions begins at a temperature at around 270°C, i.e. the thermal stability of the alkylating agent is increased by change of the anion from iodide to TFSI. The same derives from a comparison of Fig. 2c with Fig. 2d, which are thermogravimetric analyses of compounds as shown in Fig. 2a and 2b except that residue R² in these compounds is a benzyl group. In each thermogravimetric analysis, approximately 10 mg of sample were heated at 10 °C/min under a nitrogen atmosphere (20 mL/min) to 800 °C.

Subsequently, the present invention is described by means of examples, which do, however, not limit the present patent application.

### Examples 1 to 14

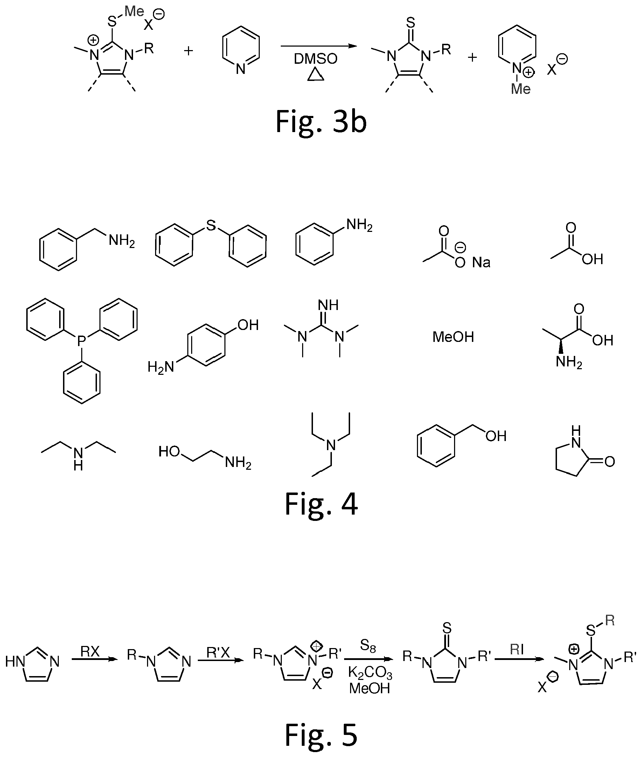

Alkylation reactions reactions were performed with the seven compounds shown in Fig. 3a, in which X⁻ is iodide, and with the seven compounds shown in Fig. 3a, in which X⁻ is TFSI. Each of the fourteen compounds was used to alkylate pyridine according the the reaction scheme shown in Fig. 3b.

For each example, a 1:1 molar ratio of alkylating agent and pyridine (0.1851 mmol) were dissolved in DMSO-d6 (0.4 mL) and added to a standard NMR tube. The sample was then heated to reaction temperature (50 or 90 °C) and analyzed *in-situ* every hour by ¹H-NMR spectroscopy for up to 13 hours. Pyridine conversion was determined by comparing the integration values of pyridine (δ = 7.99 or 7.35) to 1-methylpyridinium product (δ = 8.13), while alkylating agent conversion was determined by comparing the integration values of the thioimidazolium salt to the thione product. Second-order rate constants were obtained by plotting 1/[Pyr]ₜ as a function of time and measuring the slope. The results are shown in Table 1.

**Table 1: Rate constants for reaction of fourteen compounds with pyridine in DMSO.**

| **Compound*** | **Temperature (°C)** | **X = Iodide Rate Constant (k, M⁻¹s⁻¹)** | **X = TFSI Rate Constant (k, M⁻¹s⁻¹)** |
|---|---|---|---|
| **1** | 90 | 4.7 x 10⁻³ | No Reaction |
| **2** | 90 | 1.8 x 10⁻² | 2.6 x 10⁻⁴ |
| **3** | 90 | 4.0 x 10⁻² | 5.0 x 10⁻⁴ |
| **4** | 90 | 6.9 x 10⁻² | 5.9 x 10⁻⁴ |
| **4** | 50 | 9.8 x 10⁻⁴ | - |
| **5** | 50 | 2.3 x 10⁻³ | - |
| **6** | 50 | 9.2 x 10⁻² | 1.5 x 10⁻⁴ |
| **7** | 50 | 3.0 x 10⁻¹ | 4.9 x 10⁻⁴ |

### Examples 15 to 44

The methylation of compounds shown in Fig. 4 was conducted in either DMSO-*d6* (examples 15 to 29) or D₂O (examples 30 to 44).

### Reactions in DMSO-d6

Compound 2-I (20 mg, 0.074 mmol) was combined with 1 eq. of each of the substrates shown in Fig. 4 and DMSO-*d6* (0.5 mL) in an NMR tube. After 24 hours the reaction mixture was analyzed by ¹H-NMR spectroscopy to determine whether methylation proceeded. Conversion values were determined by comparing the integration values of the N-C*H*₃ functionality on 2-I (δ = 3.901) to N-C*H*₃ on the 1,3-dimethylimidazolethione byproduct (δ = 3.448) after alkylation. Conversions were generally low, so the reactions were heated to first 50 °C and then 80 °C at 24 hour intervals, and analyzed by ¹H-NMR spectroscopy prior to increasing temperature.

It was found that 2-I is stable at these temperatures in DMSO, so that any decomposition that occurs must be a result of nucleophilic substitution

### Reactions in D₂O

The same reactions were performed in D20. The procedures were similar to the those in DMSO-d6, except the reactions were conducted in small vials with magnetic stirring bars.

The conversion data of all these examples are shown in table 2.

**Table 2: Reaction of compound 2-I with various nucleophiles for 24 hrs at different temperatures in DMSO-d6. Reported conversions are %consumed alkylator for a given nucleophile.**

| | **RT (%)** | **50 °C (%)** | **80 °C (%)** |
|---|---|---|---|
| **1,1,3,3-tetramethylguanidine** | 1 | 23 | 98 |
| **2-pyrrolidone** | - | - | - |
| **4-aminophenol** | 1 | 16 | 74 |
| **Acetic acid, sodium salt** | - | 14 | 41 |
| **Acetic acid, glacial** | - | - | - |
| **Aniline** | - | 15 | 76 |
| **Benzyl amine** | - | 27 | 97 |
| **Benzyl alcohol** | - | - | - |
| **Diethylamine** | 2 | 37 | 99 |
| **Diphenyl sulfide** | - | <1 | 3 |
| **Ethanolamine** | <1 | 31 | 96 |
| **L-alanine** | - | 4 | 25 |
| **Methanol** | - | - | - |
| **Triethylamine** | 8 | 39 | 94 |
| **Triphenyl phosphine** | 1 | 19 | 88 |

### Synthesis of alkylating agents for formula (1) to (3)

Synthesis of all substances used in the examples followed a general procedure (Fig. 5). **1** was synthesized from tetramethylthiourea, which is commercially available and used as received.

### Step 1: Quaternization

Briefly, the parent amine (1-methylimidazole, 1-phenylimidazole, 1-(2-chlorophenyl)imidazole, or 1-methylbenzimidazole; 25 mmol) was quaternized with 2 eq Mel or BzCI (50 mmol) in acetonitrile (100 mL) at room temperature for 24 hours. The solution was then concentrated (30 mL), precipitated in diethylether (500 mL), and volatiles evaporated *in-vacuo* to isolate the iodide/chloride salts as an off-white powder in good yield (80%). Caffeine was quaternized with 4 eq. of Mel (100 mmol) in acetonitrile (100 mL) at 80 °C for 48 hours (70%).

### Step 2: Sulfurization

All quaternized salts (10 mmol) were dissolved in MeOH (40 mL) followed by the addition of 2 eq. of elemental sulfur (20 mmol) and stirred for 10 minutes. Once a suspension was obtained, 2 eq. of potassium carbonate (20 mmol) was added and left stirring for up to 48 hours. MeOH was then fully evaporated *in-vacuo* and the residue rinsed with water (3x50 mL) and recrystallized from isopropanol and dried *in-vacuo* to isolate off-white crystals (60%).

### Step 3: S-alkylation

The sulfurized product (5 mmol) was dissolved in acetonitrile (30 mL) followed by the addition of 2 eq. alkyliodide (10 mmol) and stirred at room temperature for up to 48 hours. Volatiles were removed *in-vacuo* at no more than 50 °C to isolate the S-alkylated iodide salts in quantitative yields.

### Step 4: Anion-exchanqe with TFSI

The S-alkylated iodide salts displayed variable solubility properties in water depending on the alkyliodide used for S-alkylation, with n-octyl and n-dodecyl salts displaying low solubilities in water, but excellent solubility in dichloromethane. As a result, two procedures were developed for the anion exchange reaction depending on their solubilities

### In water:

S-alkylated iodide salts (4 mmol) were dissolved in water (15 mL) followed by the addition of a slight excess of LiTFSI (4.1 mmol). This resulted in the formation of a precipitate or a biphasic system, which was stirred for 24 hours before rinsing with water (3x5 mL), until the rinsing solutions passed the silver nitrate test. The product was then dried *in-vacuo* to isolate the TFSI salts as either a liquid, or in some cases a solid (**6** and **7,** 60%).

### In dichloromethane:

S-alkylated iodide salts with either a n-octyl or n-dodecyl appendages (4 mmol) were dissolved in dichloromethane (10 mL) followed by the addition of solid LiTFSI (4 mmol). The slurry was stirred for 24 hours before filtering off the lithium iodide by gravity filtration. The solution was then rinsed with water (3x5 mL) and then dried *in-vacuo* to isolate the TFSI salts (60%).

### Compound information

### 2-I-Me

¹H-NMR (400 MHz, DMSO-d6): δ = 2.52 (s, 3H), 3.90 (s, 6H), 7.91 (s, 2H). ¹³C{¹H}-NMR (100 MHz, DMSO-d6): δ = 17.01 (s), 36.10 (s), 124.66 (s), 140.73 (s).

### 2-I-Et

¹H-NMR (400 MHz, DMSO-d6): δ = 1.17 (t, 3H, ¹*J* = 7.4 Hz), 3.05 (q, 2H, ¹*J* = 7.6 Hz), 3.91 (s, 6H), 7.95 (s, 2H). ¹³C{¹H}-NMR (100 MHz, DMSO-d6): δ = 15.13 (s), 29.64 (s), 36.23 (s), 124.96 (s), 139.48 (s).

### 2-I-Bu

¹H-NMR (400 MHz, DMSO-d6): δ = 0.87 (t, 3H, ¹*J* = 7.2 Hz), 1.36 (m, 2H), 1.48 (m, 2H), 3.02 (t, 2H, ¹*J* = 7.4 Hz), 3.90 (s, 6H), 7.93 (s, 2H). ¹³C{¹H}-NMR (100 MHz, DMSO-d6): δ = 13.30 (s), 20.95 (s), 31.46 (s), 34.57 (s), 36.19 (s), 124.93 (s), 139.63 (s).

### 2-I-Hex

¹H-NMR (400 MHz, DMSO-d6): δ = 0.85 (t, 3H, ¹*J* = 7 Hz), 1.25 (m, 4H), 1.33 (m, 2H), 1.49 (m, 2H), 3.02 (t, 2H, ¹*J* = 7.4 Hz), 3.90 (s, 6H). ¹³C{¹H}-NMR (100 MHz, DMSO-d6): δ = 13.81 (s), 21.88 (s), 27.38 (s), 29.43 (s), 30.53 (s), 34.88 (s), 36.21 (s), 124.91 (s), 139.71 (s).

### 2-I-Oct

¹H-NMR (400 MHz, DMSO-d6): δ = 0.85 (t, 3H, ¹*J* = 6.8 Hz), 1.23 (m, 8H), 1.32 (m, 2H), 1.49 (m, 2H), 3.01 (t, 2H, ¹*J* = 7.4 Hz), 3.89 (s, 6H), 7.93 (s, 2H). ¹³C{¹H}-NMR (100 MHz, DMSO-d6): δ = 13.90 (s), 21.98 (s), 27.73 (s), 28.30 (s), 28.48 (s), 29.48 (s), 31.10 (s), 34.85 (s), 36.20 (s), 124.92 (s), 139.72 (s).

### 2-I-Dodecyl

¹H-NMR (400 MHz, DMSO-d6): δ = 0.85 (t, 3H, ¹*J* = 7 Hz), 1.23 (m, 16H), 1.32 (m, 2H), 1.49 (m, 2H), 3.02 (t, 2H, ¹*J* = 7.4 Hz), 3.90 (s, 6H), 7.94 (s, 2H). ¹³C{¹H}-NMR (100 MHz, DMSO-d6): δ = 13.89 (s), 22.03 (s), 27.73 (s), 28.34 (s), 28.64 (s), 28.82 (s), 28.88 (s), 28.94 (s), 29.47 (s), 31.22 (s), 34.87 (s), 36.21 (s), 124.90 (s), 139.71 (s).

### 2-TFSI-Me

¹H-NMR (400 MHz, DMSO-d6): δ = 2.51 (s, 3H), 3.90 (s, 6H), 7.88 (s, 2H). ¹³C{¹H}-NMR (100 M, DMSO-d6): δ = 16.82 (s), 36.01 (s), 119.42 (q, ¹*J* = 319.5 Hz), 124.71 (s), 140.75 (s).

### 2-TFSI-Et

¹H-NMR (400 MHz, DMSO-d6): δ = 1.17 (t, 3H, ¹*J* = 7.4 Hz), 3.04 (q, 2H, ¹*J* = 7.2 Hz), 3.90 (s, 6H), 7.92 (s, 2H). ¹³C{¹H}-NMR (100 MHz, DMSO-d6): δ = 15.10 (s), 29.58 (s), 36.16 (s), 119.41 (q, ¹*J* = 319.9 Hz), 125.01 (s), 139.50 (s).

### 2-TFSI-Bu

¹H-NMR (400 MHz, DMSO-d6): δ = 0.88 (t, 3H, ¹*J* = 7.2 Hz), 1.36 (m, 2H), 1.49 (m, 2H), 3.01 (t, 2H, ¹*J* = 7.2 Hz), 3.90 (s, 6H), 7.91 (s, 2H). ¹³C{¹H}-NMR (100 MHz, DMSO-d6): δ = 13.26 (s), 20.97 (s), 31.48 (s), 34.54 (s), 36.13 (s), 119.42 (q, ¹*J* = 319.9 Hz), 124.96 (s), 139.72 (s).

### 2-TFSI-Oct

¹H-NMR (400 MHz, DMSO-d6): δ = 0.85 (t, 3H, ¹*J* = 6.8 Hz), 1.25 (m, 4H), 1.33 (m, 2H), 1.50 (m, 2H), 3.01 (t, 2H, ¹*J* = 7.4 Hz), 3.89 (s, 6H). ¹³C{¹H}-NMR (100 MHz, DMSO-d6): δ = 13.80 (s), 21.90 (s), 27.42 (s), 29.46 (s), 30.57 (s), 34.82 (s), 36.14 (s), 119.41 (q, ¹*J* = 319.8 Hz), 124.96 (s), 139.73 (s).

### 2-TFSI-Dodecyl

¹H-NMR (400 MHz, DMSO-d6): δ = 0.85 (t, 3H, ¹*J* = 6.8 Hz), 1.24 (m, 8H), 1.33 (m, 2H), 1.50 (m, 2H), 3.01 (t, 2H, ¹*J* = 7.4 Hz), 3.89 (s, 6H), 7.91 (s, 2H). ¹³C{¹H}-NMR (100 MHz, DMSO-d6): δ = 13.87 (s), 22.00 (s), 27.76 (s), 28.32 (s), 28.50 (s), 29.49 (s), 31.13 (s), 34.84 (s), 36.13 (s), 119.41 (q, ¹*J* = 319.9 Hz), 124.95 (s), 139.73 (s).

### 3-I-Me

¹H-NMR (400 MHz, DMSO-d6): δ = 2.38 (s, 3H), 3.93 (s, 3H), 5.54 (s, 2H), 7.38 (m, 5H), 8.00 (m, 2H). ¹³C{¹H}-NMR (100 M, DMSO-d6): δ = 17.36 (s), 36.20 (s), 52.06 (s), 124.02 (s), 125.55 (s), 127.70 (s), 128.46 (s), 128.90 (s), 134.76 (s), 140.70 (s).

### 3-I-Et

¹H-NMR (400 MHz, DMSO-d6): δ = 1.11 (m, 3H), 2.94 (m, 2H), 3.94 (s, 3H), 5.56 (s, 2H), 7.38 (m, 5H), 8.05 (m, 2H). ¹³C{¹H}-NMR (100 MHz, DMSO-d6): δ = 14.77 (s), 30.11 (s), 36.43 (s), 52.08 (s), 124.26 (s), 125.78 (s), 127.71 (s), 128.47 (s), 128.85 (s), 134.67 (s), 139.53 (s).

### 3-I-Bu

¹H-NMR (400 MHz, DMSO-d6): δ = 0.80 (t, 3H, ¹*J* = 7.2 Hz), 1.25 (m, 2H), 1.40 (m, 2H), 3.93 (s, 3H), 5.55 (s, 2H), 7.37 (m, 5H), 8.04 (m, 2H). ¹³C{¹H}-NMR (100 MHz, DMSO-d6): δ = 13.25 (s), 21.01 (s), 31.16 (s), 34.97 (s), 36.41 (s), 52.08 (s), 124.31 (s), 125.73 (s), 127.60 (s), 128.43 (s), 128.85 (s), 134.72 (s), 139.74 (s).

### 3-I-Hex

¹H-NMR (400 MHz, DMSO-d6): δ = 0.83 (t, 3H, ¹*J* = 7 Hz), 1.20 (m, 6H), 1.40 (m, 2H), 2.85 (t, 2H, ¹*J* = 7.6 Hz), 3.93 (s, 3H), 5.55 (s, 2H), 7.37 (m, 5H), 8.78 (m, 2H). ¹³C{¹H}-NMR (100 MHz, DMSO-d6): δ = 13.80 (s), 21.83 (s), 27.43 (s), 29.13 (s), 30.47 (s), 35.23 (s), 36.40 (s), 52.07 (s), 124.32 (s), 125.73 (s), 127.57 (s), 128.43 (s), 128.85 (s), 134.73 (s), 139.77 (s).

### 3-I-Oct

¹H-NMR (400 MHz, DMSO-d6): δ = 0.85 (t, 3H, ¹*J* = 7 Hz), 1.18 (m, 10H), 1.40 (m, 2H), 2.85 (t, 2H, ¹*J* = 7.6 Hz), 3.92 (s, 3H), 5.54 (s, 2H), 7.37 (m, 5H), 8.04 (m, 2H). ¹³C{¹H}-NMR (100 MHz, DMSO-d6): δ = 13.91 (s), 21.97 (s), 27.77 (s), 28.22 (s), 28.42 (s), 29.17 (s), 31.08 (s), 35.22 (s), 36.39 (s), 52.07 (s), 124.32 (s), 125.73 (s), 127.57 (s), 128.42 (s), 128.84 (s), 134.73 (s), 139.78 (s).

### 3-I-Dodecyl

¹H-NMR (400 MHz, DMSO-d6): δ = 0.85 (t, 3H, ¹*J* = 6.8 Hz), 1.23 (m, 18H), 1.40 (m, 2H), 2.85 (t, 2H, ¹*J* = 7.6 Hz), 3.92 (s, 3H), 5.54 (s, 2H), 7.36 (m, 5H), 8.04 (m, 2H). ¹³C{¹H}-NMR (100 MHz, DMSO-d6): δ = 13.921 (s), 22.05 (s), 27.77 (s), 28.27 (s), 28.66 (s), 28.77 (s), 28.87 (s), 28.95 (s), 29.18 (s), 31.24 (s), 35.23 (s), 36.39 (s), 52.07 (s), 124.31 (s), 125.73 (s), 127.58 (s), 128.41 (s), 128.83 (s), 134.73 (s), 139.77 (s).

### 3-TFSI-Me

¹H-NMR (400 MHz, DMSO-d6): δ = 2.38 (s, 3H), 3.94 (s, 3H), 5.55 (s, 2H), 7.39 (m, 5H), 7.99 (m, 2H). ¹³C{¹H}-NMR (100 M, DMSO-d6): δ = 17.22 (s), 36.11 (s), 52.11 (s), 119.44 (q, ¹*J* = 319.8 Hz), 124.04 (s), 125.57 (s), 127.69 (s), 128.46 (s), 128.88 (s), 134.72 (s), 140.71 (s).

### 3-TFSI-Et

¹H-NMR (400 MHz, DMSO-d6): δ = 2.38 (s, 3H), 3.94 (s, 3H), 5.55 (s, 2H), 7.39 (m, 5H), 7.99 (m, 2H). ¹³C{¹H}-NMR (100 MHz, DMSO-d6): δ = 17.22 (s), 36.11 (s), 52.11 (s), 119.44 (q, ¹*J* = 319.9 Hz), 124.04 (s), 125.57 (s), 127.69 (s), 128.46 (s), 128.88 (s), 134.72 (s), 140.71 (s).

### 3-TFSI-Bu

¹H-NMR (400 MHz, DMSO-d6): δ = 0.81 (t, 3H, ¹*J* = 7.4 Hz), 1.26 (m, 2H), 1.41 (m, 2H), 2.50 (s, 2H), 2.86 (t, 2H, ¹*J* = 7.4 Hz), 3.93 (s, 6H), 5.54 (s, 2H), 7.37 (m, 5H), 8.02 (m, 2H). ¹³C{¹H}-NMR (100 MHz, DMSO-d6): δ = 13.17 (s), 21.00 (s), 31.16 (s), 34.92 (s), 36.30 (s), 52.11 (s), 119.42 (q, ¹*J* = 319.9 Hz), 124.34 (s), 125.75 (s), 127.58 (s), 128.44 (s), 128.85 (s), 134.71 (s), 139.76 (s).

### 3-TFSI-Hex

¹H-NMR (400 MHz, DMSO-d6): δ = 0.84 (t, 3H, ¹*J* = 7 Hz), 1.20 (m, 6H), 1.42 (m, 2H), 2.85 (m, 2H), 3.93 (s, 3H), 5.55 (s, 2H), 7.37 (m, 5H), 8.02 (m, 2H). ¹³C{¹H}-NMR (100 MHz, DMSO-d6): δ = 13.71 (s), 21.82 (s), 27.44 (s), 29.14 (s), 30.47 (s), 35.19 (s), 36.29 (s), 52.11 (s), 119.42 (q, ¹*J* = 319.9 Hz), 124.34 (s), 125.74 (s), 127.55 (s), 128.42 (s), 128.83 (s), 134.71 (s), 139.79 (s).

### 3-TFSI-Oct

¹H-NMR (400 MHz, DMSO-d6): δ = 0.85 (t, 3H, ¹*J* = 7 Hz), 1.19 (m, 10H), 1.42 (m, 2H), 2.85 (t, 2H, ¹*J* = 7.4 Hz), 3.92 (s, 3H), 5.54 (s, 2H), 7.37 (m, 5H), 8.02 (m, 2H). ¹³C{¹H}-NMR (100 MHz, DMSO-d6): δ = 13.84 (s), 21.97 (s), 27.77 (s), 28.22 (s), 28.42 (s), 29.17 (s), 31.09 (s), 35.18 (s), 36.30 (s), 52.09 (s), 119.41 (q, ¹*J* = 319.9 Hz), 124.33 (s), 125.74 (s), 127.55 (s), 128.42 (s), 128.83 (s), 134.71 (s), 139.79 (s).

### 3-TFSI-Dodecyl

¹H-NMR (400 MHz, DMSO-d6): δ = 0.85 (t, 3H, ¹*J* = 7 Hz), 1.23 (m, 18H), 1.41 (m, 2H), 2.85 (t, 2H, ¹*J* = 7.4 Hz), 3.92 (s, 3H), 5.54 (s, 2H), 7.37 (m, 5H), 8.02 (m, 2H). ¹³C{¹H}-NMR (100 MHz, DMSO-d6): δ = 13.91 (s), 22.07 (s), 27.79 (s), 28.28 (s), 28.68 (s), 28.79 (s), 28.88 (s), 28.96 (s), 28.97 (s), 29.19 (s), 31.26 (s), 35.19 (s), 36.33 (s), 52.09 (s), 119.41 (q, ¹*J* = 319.9 Hz), 124.34 (s), 125.75 (s), 127.57 (s), 128.44 (s), 128.85 (s), 134.75 (s), 139.79 (s).

### 4-I

¹H-NMR (400 MHz, DMSO-d6): δ = 2.34 (s, 3H, S-C*H*₃), 4.00 (s, 3H), 7.67 (m, 5H), 8.13 (s, 1H), 8.17 (s, 1H). ¹³C-NMR (100 MHz, DMSO-d6): δ = 17.2 (s), 36.3 (s), 125.0 (s), 125.1 (s), 126.3 (s), 129.7 (s), 130.5 (s), 135.3 (s), 141.8 (s).

### 4-TFSI

¹H-NMR (400 MHz, DMSO-d6): δ = 2.33 (s, 3H, S-C*H*₃), 4.00 (s, 3H), 7.67 (m, 5H), 8.12 (s, 1H), 8.17 (s, 1H). ¹³C{¹H}-NMR (100 MHz, DMSO-d6): δ = 17.2 (s), 36.3 (s), 125.0 (s), 125.1 (s), 126.3 (s), 129.7 (s), 130.5 (s), 135.3 (s), 141.8 (s). ¹³C-NMR (100 MHz, DMSO-d6): δ = 17.1 (s), 36.3 (s), 119.4 (q, ¹J_{C-F} = 320 Hz), 125.1 (s), 125.2 (s), 126.3 (s), 129.7 (s), 130.6 (s), 135.3 (s), 141.8 (s).

### 5-I

¹H-NMR (400 MHz, DMSO-d6): δ = 2.40 (s, 3H, S-C*H*₃), 4.00 (s, 3H), 7.67 (m, 2H), 7.84 (m, 2H) 8.21 (s, 1H), 8.22 (s, 1H). ¹³C-NMR (100 MHz, DMSO-d6): δ = 17.2 (s), 36.6 (s), 125.4 (s), 125.8 (s), 128.7 (s), 129.8 (s), 130.1 (s), 130.4 (s), 132.6 (s), 132.9 (s), 142.5 (s).

### 6-I

¹H-NMR (400 MHz, DMSO-d6): δ = 2.72 (s, 3H, S-C*H*₃), 4.13 (s, 6H), 7.72 (m, 2H), 8.06 (m, 2H). ¹³C-NMR (100 MHz, DMSO-d6): δ = 17.1 (s), 33.2 (s), 113.2 (s), 126.7 (s), 132.1 (s), 149.9 (s).

### 6-TFSI

¹H-NMR (400 MHz, DMSO-d6): δ = 2.71 (s, 3H, S-C*H*₃), 4.13 (s, 6H), 7.72 (m, 2H), 8.07 (m, 2H). ¹⁹F-NMR (376 MHz, DMSO-d6): δ = -78.8 (s) ¹³C-NMR (100 MHz, DMSO-d6): δ = 17.0 (s), 33.1 (s), 113.2 (s), 119.4 (q, ¹J_{C-F} = 320 Hz), 126.8 (s), 132.1 (s), 149.9 (s).

### 7-I

¹H-NMR (400 MHz, DMSO-d6): δ = 2.59 (s, 3H, S-C*H*₃), 3.29 (s, 3H), 3.79 (s, 3H), 4.19 (s, 3H), 4.23 (s, 3H). ¹³C-NMR (100 MHz, DMSO-d6): δ = 28.0 (s), 31.7 (s), 33.3 (s), 34.2 (s), 103.9 (s), 139.1 (s), 150.3 (s), 152.2 (s), 165.6 (s).

### 7-TFSI

¹H-NMR (400 MHz, DMSO-d6): δ = 2.59 (s, 3H, S-C*H*₃), 3.29 (s, 3H), 3.79 (s, 3H), 4.19 (s, 3H), 4.23 (s, 3H). ¹³C-NMR (100 MHz, DMSO-d6): δ = 17.5 (s), 28.5 (s), 32.1 (s), 35.6 (s), 36.3 (s), 108.6 (s), 119.4 (q, ¹J_{C-F} = 320 Hz) 140.0 (s), 145.8 (s), 150.0 (s), 152.9 (s).

### Synthesis of alkylating agents for formula (4)

Synthesis proceeded using a modified Debus-Radiziszewski reaction. Briefly, methylamine (40 wt% in water, 0.40 g, 12.88 mmol) was added to acetic acid (0.90 g, 15.0 mmol) in a vial dropwise at 0 °C and then warmed to room temperature. In a separate vial, 4-(methylthio)benzaldehyde (1.08 g, 7.08 mmol) was combined with glyxoal (40 wt% in water, 0.748 g, 12.88 mmol), 2 mL isopropanol, and 1.25 mL acetic aicd. The two vials were then combined quickly and left stirring for 24 hours, after which the solution was extracted with ethyl acetate (3x50 mL) and a 5 mL aqueous solution of LiTFSI (4.0 g, 14.0 mmol) added to the reaction mixture. A brown oil was formed upon addition and was rinsed with water (3x10 mL) and dried *in-vacuo* to isolate the product.

## Claims

1. Method for alkylating a molecule or an ion, wherein
the molecule or the ion is alkylated by reacting it with an alkylating agent, wherein
the alkylating agent is a compound according to formula (1): 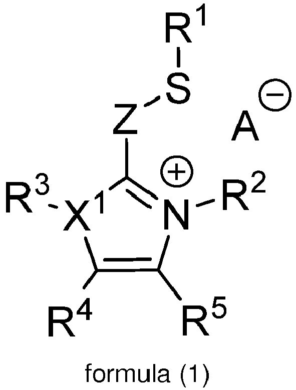 wherein in formula (1)
R¹ is a substituted or unsubstituted C₁₋₂₀ hydrocarbon residue or a polymer,
R² is a substituted or unsubstituted C₁₋₂₀ hydrocarbon residue or a hydrogen atom,
R³ is absent, a substituted or unsubstituted C₁₋₂₀ hydrocarbon residue or a hydrogen atom,
R⁴ and R⁵ are linked with each other to form an aromatic group or are independently from each other selected from the group consisting of a hydrogen atom, substituted and unsubstituted C₁₋₂₀ hydrocarbon residues and electron withdrawing groups,
X¹ is an oxygen atom, a nitrogen atom or a sulfur atom, wherin R³ is not present in formula (1) if X¹ is an oxygen atom or a sulfur atom,
Z is a covalent bond or an aromatic group and
A⁻ is an anion.

2. Method according to claim 1, wherein the alkylating agent is a compound according to formula (2): 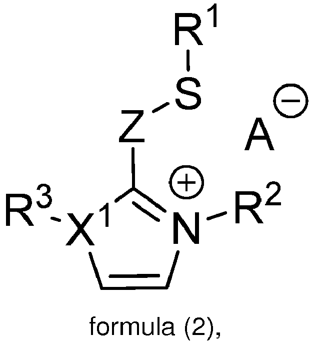 wherein R¹, R², R³, X¹, Z and A⁻ are defined as in claim 1.

3. Method according to claim 2, wherein
Z is a covalent bond and
X¹ is a nitrogen atom.

4. Method according to any of the preceding claims, wherein
R² is equal to R³,
Z is a covalent bond and
X¹ is a nitrogen atom

5. Method according to any of the preceding claims, wherein R¹ is a substituted or unsubstituted C₁₋₁₂ alkyl group, a substituted or unsubstituted C₄₋₁₂ cycloalkyl group, a substituted or unsubstituted C₂₋₁₂ alkenyl group or a substituted or unsubstituted C₂₋₁₂ alkinyl group.

6. Method according to claim 1, wherein
the alkylating agent is a compound according to formula (3): 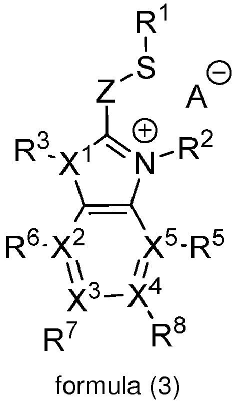 wherein in formula (3)
R¹ is a substituted or unsubstituted C₁₋₂₀ hydrocarbon residue or a polymer,
R² is a substituted or unsubstituted C₁₋₂₀ hydrocarbon residue or a hydrogen atom,
R³ is absent, a substituted or unsubstituted C₁₋₂₀ hydrocarbon residue or a hydrogen atom,
R⁶, R⁷, R⁸ and R⁹ are independently from each other selected from the group consisting of a hydrogen atom, an oxygen atom, a sulfur atom, substituted or unsubstituted C₁₋₂₀ hydrocarbon residues and electron withdrawing groups, and/or any of R⁶, R⁷, R⁸ and R⁹ is linked to another one of R⁶, R⁷, R⁸ and R⁹ to form a cyclic structure,
X¹ is an oxygen atom, a nitrogen atom or a sulfur atom, wherein R³ is not present in formula (3) if X¹ is an oxygen atom or a sulfur atom,
X², X³, X⁴ and X⁵ are independently from each other selected from the group consisting of a carbon atom, a nitrogen atom, an oxygen atom and a sulfur atom, and/or X², X³, X⁴ and X⁵ form an aromatic ring and
A⁻ is an anion.

7. Method according to claim 6, wherein
X¹ is a nitrogen atom and
X², X³, X⁴ and X⁵ are carbon atoms.

8. Method according to claim 1, wherein the alkylating agent is a compound according to formula (4): 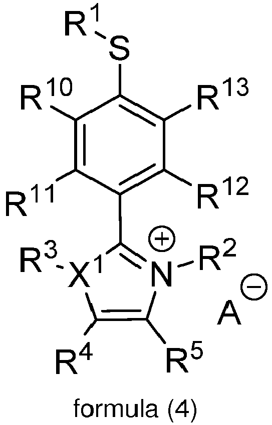 wherein in formula (4)
R¹ is a substituted or unsubstituted C₁₋₂₀ hydrocarbon residue or a polymer,
R² is a substituted or unsubstituted C₁₋₂₀ hydrocarbon residue or a hydrogen atom,
R³ is absent, a substituted or unsubstituted C₁₋₂₀ hydrocarbon residue or a hydrogen atom,
R⁴ and R⁵ are linked with each other to form an aromatic group or are independently from each other selected from the group consisting of a hydrogen atom, substituted and unsubstituted C₁₋₂₀ hydrocarbon residues and electron withdrawing groups,
R¹⁰ to R¹³ are independently selected from the group consisting of hydrogen, substituted and unsubstituted C₁₋₂₀ hydrocarbon residues and electron withdrawing groups,
X¹ is an oxygen atom, a nitrogen atom or an sulfur atom, wherein R³ is not present in formula (4) if X¹ is an oxygen atom or a sulfur atom, and A⁻ is an anion.

9. Method according to claim 8, wherein
R¹⁰ to R¹³ are hydrogen atoms and
X¹ is a nitrogen atom.

10. Method according to claim 8, wherein
R¹ is a substituted or unsubstituted C₁₋₂₀ alkyl group, a substituted or unsubstituted C₃₋₂₀ cycloalkyl group, a substituted or unsubstituted C₂₋₂₀ alkenyl group or a substituted or unsubstituted C₂₋₂₀ alkinyl group,
at least one of R⁴, R⁵, R¹⁰, R¹¹, R¹², and R¹³ is an electron withdrawing group which is selected from the group consisting of carbonyl groups, hydroxyl groups, halogen atoms, carboxy groups, cyano groups fluorinated and perfluorinated alkyl groups,
X¹ is a nitrogen atom and
X², X³, X⁴ and X⁵ are carbon atoms.

11. Method according to any of the preceding claims, wherein
the anion is selected from the group consisting of TFSI, BR¹⁴₄⁻, PF₆⁻ and B(C₆H₅)₄⁻, wherein in BR¹⁴₄⁻ R¹⁴ is selected from the group consisiting of substituted or unsubstituted C₁₋₁₀ hydrocarbon residues and halogen atoms.

12. Compound according to formula (1a): 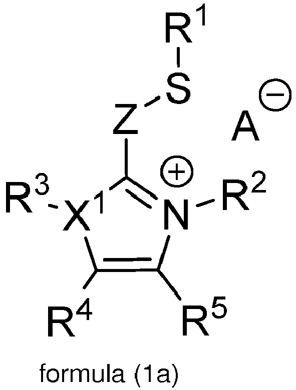 wherein in formula (1a)
R¹ is a substituted or unsubstituted C₁₋₂₀ hydrocarbon residue or a polymer, R² is a substituted or unsubstituted C₁₋₂₀ hydrocarbon residue or a hydrogen atom,
R³ is absent, a substituted or unsubstituted C₁₋₂₀ hydrocarbon residue or a hydrogen atom,
R⁴ and R⁵ are linked with each other to form an aromatic group or are independently from each other selected from the group consisting of a hydrogen atom, substituted and unsubstituted C₁₋₂₀ hydrocarbon residues and electron withdrawing groups,
X¹ is an oxygen atom, a nitrogen atom or a sulfur atom, wherin R³ is not present in formula (1a) if X¹ is an oxygen atom or a sulfur atom,
Z is a covalent bond or an aromatic group and
A⁻ is an anion which is not alkylated to a degree of more than 10% when being in contact with methyl iodide for 10 minutes at room temperature, at atmospheric pressure in a molar ratio of A⁻ to methyl iodide of 1:1.

13. Compound according to claim 12, wherein
A⁻ is an anion containing at least one of a fluorinated hydrocarbon residue, a perfluorinated hydrocarbon residue, and/or an anionic charge centred about a borate, phosphate, phosphonate, sulfate, sulfonate, acetate, perchlorate, triazolate, and/or cyanamide structure.

14. Compound according to claim 13, wherein
the anion is selected from the group consisting of BR¹⁴₄⁻, PF₆⁻, B(C₆H₅)₄⁻, triflate, TFSI, FSI, BETI, TFSAM, TSAC and DCA, wherein
in BR¹⁴₄⁻ R¹⁴ is a moiety selected from the group consisiting of substituted or unsubstituted C₁₋₂₀ hydrocarbon residues and halogen atoms.

15. Compound according to claim 14, wherein
the anion is selected from the group consisting of TFSI, BR¹⁴₄⁻, PF₆⁻ and B(C₆H₅)₄, wherein in BR¹⁴₄⁻ R¹⁴ is selected from the group consisiting of substituted or unsubstituted C₁₋₁₀ hydrocarbon residues and halogen atoms.
